Europäisches Patentamt

European Patent Office

Office européen des brevets

(11) Publication number: **0 380 966**
**A1**

## EUROPEAN PATENT APPLICATION

(21) Application number: **90100928.2**

(51) Int. Cl.⁵: **G06F 15/42**

(22) Date of filing: **17.01.90**

(30) Priority: **30.01.89 US 303861**

(43) Date of publication of application:
**08.08.90 Bulletin 90/32**

(84) Designated Contracting States:
**DE FR GB**

(71) Applicant: **Siemens Aktiengesellschaft**
**Wittelsbacherplatz 2**
**D-8000 München 2(DE)**

(72) Inventor: **Prior, Fred W.**
**5779 Essex Court**
**Hanover Park, IL 60103(US)**
Inventor: **Nabijee, Kamal H.**
**565 Heritage Drive**
**No. 109, Hoffman Estates, IL 60194(US)**

(54) **Information management system, particularly for use in picture archive and communications systems.**

(57) An information management system stores data objects produced by diagnostic apparatus on at least one mass storage device. The mass storage devices are addressed by a single database server, which stores data which is not data objects.

FIG 1

## BACKGROUND OF THE INVENTION

The invention relates to information management, and more particularly relates to information management for medical applications. In its most immediate sense, the invention relates to an information management system for use in a PACS (Picture Archive and Communications System).

A hospital or other clinical facility usually utilizes a plurality of imaging modalities. For example, a hospital may own X-ray equipment, a magnetic resonance imager, a CT scanner, ultrasound imagers and scintillation cameras. These different modalities are used individually and in combination, so that a patient may, during a particular hospital admission, undergo repeated CT scans, a magnetic resonance study, a nuclear medicine study using a scintillation camera, etc.

At present, the overwhelming majority of hospitals store the image data so acquired on photographic film. While this has proved satisfactory in the past, the volume of information involved makes this storage method unsatisfactory. It is now generally accepted that such data should, in the future, be stored electronically, in digital form, and displayed as necessary on video screens. Systems which do this are called Picture Archive and Communications Systems.

The volume of digital information produced by a fully digital radiology department has been estimated. Based on several hundred thousand images per year, information storage requirements can mount to several Terabytes per year. (A Terabyte equals $10^{12}$ bytes.)

While it is now recognized that techniques of mass storage must be improved to cope with the large volumes of data which a digital radiology department will generate, little attention has been given to the way in which this data should be organized and accessed. In some areas, hospitals must maintain this data for up to 25 years to satisfy applicable legal requirements. Consequently, the task of locating a particular image or series of images can be expected to take progressively longer times. If the PACS spends inordinate computer resources merely locating data of interest, performance of the whole system will inevitably be degraded.

It would therefore be desirable to provide a system which could not only store large quantities of data, but which could also access the data quickly and efficiently.

## SUMMARY OF THE INVENTION

One object of the invention is to provide an information management system which can quickly locate information of interest, even where large quantities (on the order of Terabytes) of information are involved.

Another object is to provide such a system which can be implemented in a PACS.

A further object is to provide such a system which can be easily used by doctors and hospital administrators in a clinical environment.

Still another object is to provide such a system which can satisfy, in a cost-effective matter, legal requirements applicable to the retention of medical information.

An additional object is to provide such a system which can cope with the open-ended growth which can be expected of data in a PACS.

The invention proceeds from the realization that it is always much easier for a computer to manipulate references to data (such references are known in the computer art as "pointers") than it is for the computer to manipulate the data itself. Thus, in accordance with the invention, an information management system separates the storage of the ultimate data from the storage and manipulation of the pointers which reference that data. The data itself (referred to hereinafter as the "data objects") may be stored on one or more mass storage devices but is excluded from a unique database server which has the function of manipulating pointers.

Advantageously, each data object (e.g. each image, each graph, each field of text, where the invention is implemented in a PACS) is uniquely identified by a data object identifier and, as stored in a mass storage unit or units, the data objects are keyed by data object identifiers. The data object identifiers, in turn, are manipulated in a single database server which only manipulates index data, and the data object identifiers are keyed using a set of data elements (hereinafter referred to as "data object descriptions") which are examined when individual data objects, or groups of data objects, are retrieved. Where the invention is implemented in a PACS, data object identifiers are, for example, patient name, modality type, date, etc.

In a preferred embodiment of the invention where the invention is implemented in a PACS, data is compressed and decompressed in such a manner as to remove data from the database server when the data need not be kept immediately available. This is accomplished by using a data structure in which data is stored in compressed form as nested data objects and stored in expanded form as database entities. The nesting structure is particularly adapted to the needs of physicians and hospital personnel.

In brief, data is put into a single data format which can reference data objects themselves, or which can reference references to data objects. When a data object contains pointers to other data objects, the pointer-containing data object is called a "folder". In the preferred embodiment, the various folders are designed to have particular utility in a PACS.

When a folder must be kept immediately available (as where it relates to a patient who is currently hospitalized and whose records must be accessed) it is stored in the database server. When the folder need not be immediately available, it is stored in the form of nested data objects in the mass storage device(s).


BRIEF DESCRIPTION OF THE DRAWINGS


Exemplary and non-limiting preferred embodiments of the invention are shown in the drawings, in which:

Fig. 1 schematically illustrates the architecture of a PACS in accordance with a preferred embodiment;

Fig. 2 schematically illustrates the data object format in accordance with the preferred embodiment of the invention;

Fig. 3 schematically illustrates the data structure of the preferred embodiment of the invention;

Figs. 4, 5 and 6 show data in various states of compression; and

Fig. 7 schematically illustrates how, in the preferred embodiment, data is compressed and decompressed.


DETAILED DESCRIPTION OF PREFERRED EMBODIMENTS


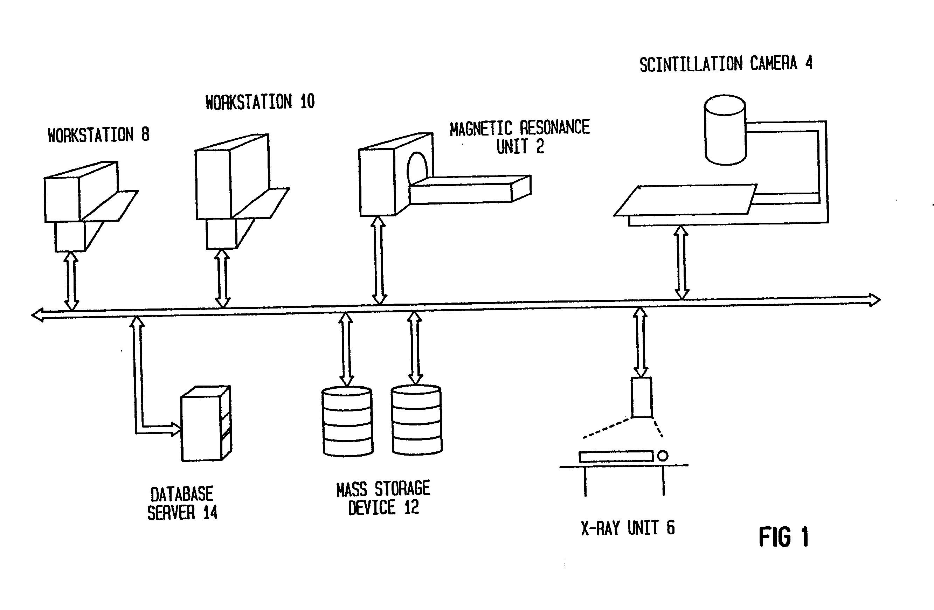

Fig. 1 shows a PACS as it might be installed in a hospital radiology department. Image data is acquired by e.g. a magnetic resonance unit 2, a scintillation camera 4 or an X-ray unit 6. This data is stored as described below. When a physician or administrator wishes to view the medical records of a patient, including text, images or both, the records may be brought up on either of the workstations 8 and 10 and updated as desired. It will be understood that Fig. 1 is schematic and neither the number and nature of imaging modalities nor the number and nature of workstations is a part of the invention.

An active radiology department will produce Terabytes of information per year. In accordance with the invention, most of this is stored on one or more mass storage devices 12. The rest is stored on a single database server 14. The manner in which this is accomplished is described below.

In accordance with the invention, the data produced by an imaging modality is always stored in the form of data objects on the mass storage device(s) 12. However, references to this data may be stored either on the mass storage device(s) 12 or on the database server 14.

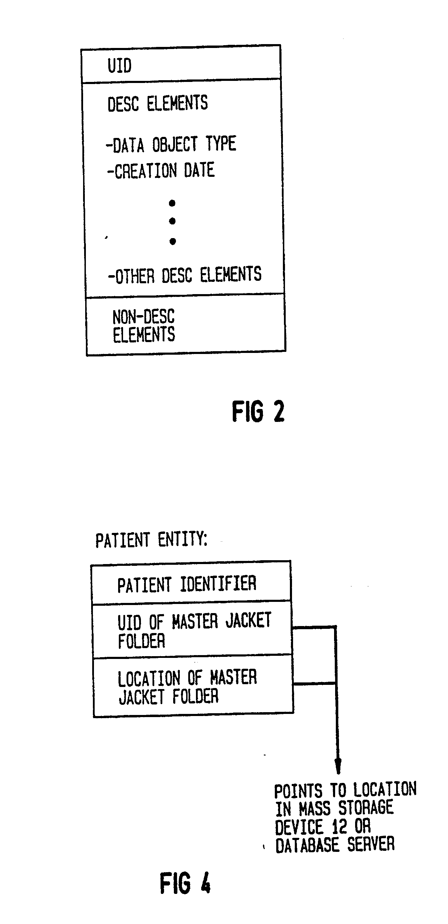

In the preferred embodiment, a basic building block is the data object format (see Fig. 2). This contains, in addition to other information:
a unique data object identifier, or UID;
a data object description, or DESC; and
non-DESC elements.

The UID is advantageously in the form defined by a Siemens Aktiengesellschaft extension to ACR-NEMA Digital Imaging and Communications Standard 300-1985. The extension, denominated Standard Product Interconnect (SPI) for Compatibility of Digital Imaging, serves to identify the location of the data involved, the equipment which generates the data, and the date and time that the data was created. In short, the UID is part of a dated "pointer". The pointer is made up of the UID plus a system address which together uniquely identify the location of an information-containing data object.

The DESC is a set of data elements which are used to select data objects or folders (described below). Examples of DESC elements are patient name, imaging modality and organ system imaged. When, for example, a physician wishes to see an image of a patient's brain, an eventual prerequisite to finding such an image is a search to find data objects which relate to that patient's name. Another example of a DESC is a "type" element; this identifies whether the data object is a folder (described below) or not.

The non-DESC elements can be either a) image data, graph data, text etc. or b) UID's. A data object might, for example, be an image of a cross-section through a patient's heart, and in this case the non-DESC elements would be the information in that image. Alternatively, the non-DESC elements might be the UID's of other data objects. As an example, a meta-data object in data object format may, in non-DESC elements, identify the UID's of all data which relates to a single examination of a patient, so that one UID identifies the location of the relevant image, another UID identifies the location of the radiologist's report, a third identifies the location of the patient's electrocardiogram, etc. If its non-DESC elements are UID's, a meta-data object in the form of a data object is called a "folder".

The preferred embodiment uses a structure which is designed to have particular utility in a radiology department. All of the ultimate data objects which relate to a single examination of the patient are contained in, and are therefore connected by, references in an Examination Folder. All of the examinations which relate to a single hospital admission are contained in, and are therefore connected by, references in a Hospitalization Folder. the patient's medical history is contained in a History Folder. The patient's demographic information is contained in a Demographics Folder, and the patient's medical history, hospitalization history and demographics information are contained in, and are therefore connected by, a Master Jacket Folder. The Master Jacket Folder is linked to a Patient Entity which identifies the patient's Master Jacket Folder even after the Master Jacket Folder has been archived (see below).

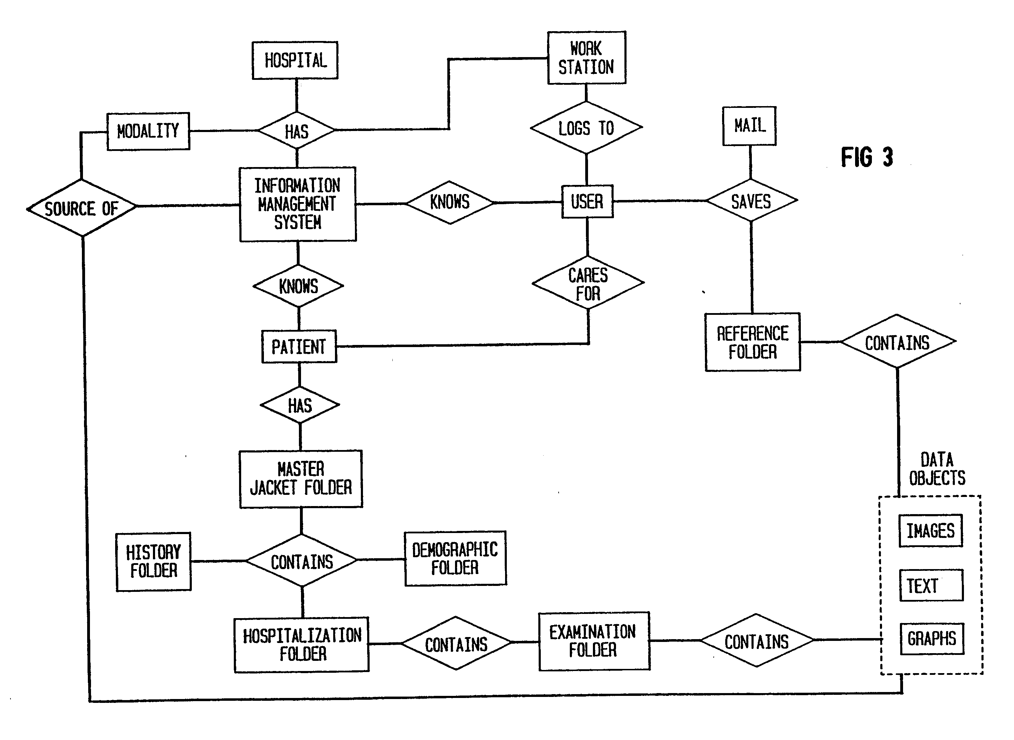

Fig. 3 is a schematic illustration of the data structure used in the preferred embodiment of the invention. In this illustration, diamonds indicate relationships and unshaded rectangles indicate data. Shaded rectangles indicate folders, as described above. The Reference Folder is a user-defined folder which allows the user to associate information of interest.

Before further describing the preferred embodiment, it is appropriate to discuss the difference between the data object format and a data object as such. In the preferred embodiment, all information is stored in a single format, which is called the data object format. If the information stored is produced by an imaging modality or by a user, the information is called a data object. If, on the other hand, the information stored is information about connections between data objects, the information is called a folder. Folders are in the same format as data objects; the only differences reside in the types of information stored in them.

Because of this identicality of data storage format, the data in the database as a whole may conveniently be expanded and compressed by transformations between folders in data object format and database entities in the database server 14. This is because there is an isomorphism between folder data objects and database entities in the database server 14. In the preferred embodiment, data objects are always stored on the mass storage device(s) 12. Folders, on the other hand, may be stored on the database server 14 or on the mass storage device(s) 12, depending on how accessible they need to be.

When a patient's records must be immediately accessible (as where the patient is currently hospitalized), all that patient's folders are stored in the database server 14 as database entities. When a patient's records must be accessible but need not be immediately accessible (as where the patient was admitted in the past year), the master jacket is stored in the database server 14 as a database entity but all lower-level folders and data objects are stored in nested data object format on the mass storage device(s) 12. When, on the other hand, the patient's records need only be locatable (as where the patient was last hospitalized more than one year previously), the master jacket and all the other folders to which it relates are archived in nested data object format and only the Patient Entity remains in the database server 14, to identify the location of the Master Jacket Folder.

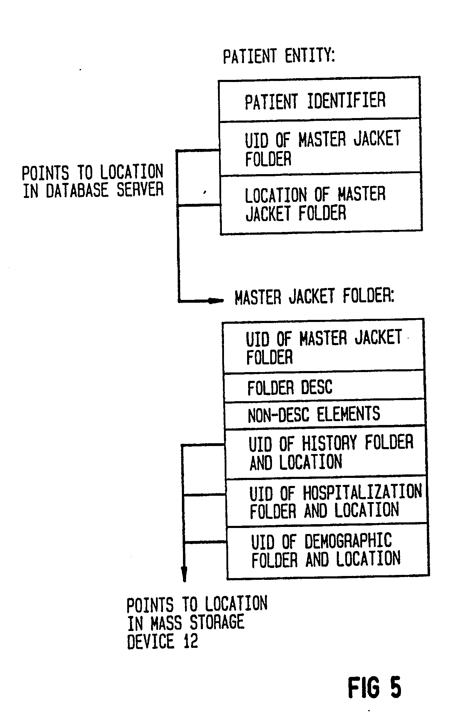

Thus, as can be seen in Fig. 4, when for example a patient is currently hospitalized, the Patient Entity, Master Jacket Folder, History Folder, Demographics Folder, Hospitalization Folder and all the Examination Folders are all resident in the database server 14. It is thus possible to retrieve all the information which relates to the patient very quickly.

On the other hand, as is shown in Fig. 5, when for example a patient has been hospitalized within the last year, the Patient Entity and Master Jacket Folder are both resident in the database server 14, but the History Folder, Demographics Folder, Hospitalization Folder and all the Examination Folders reside in the mass storage device(s) 12. It thus takes a longer time to retrieve all the information which relates to the patient.

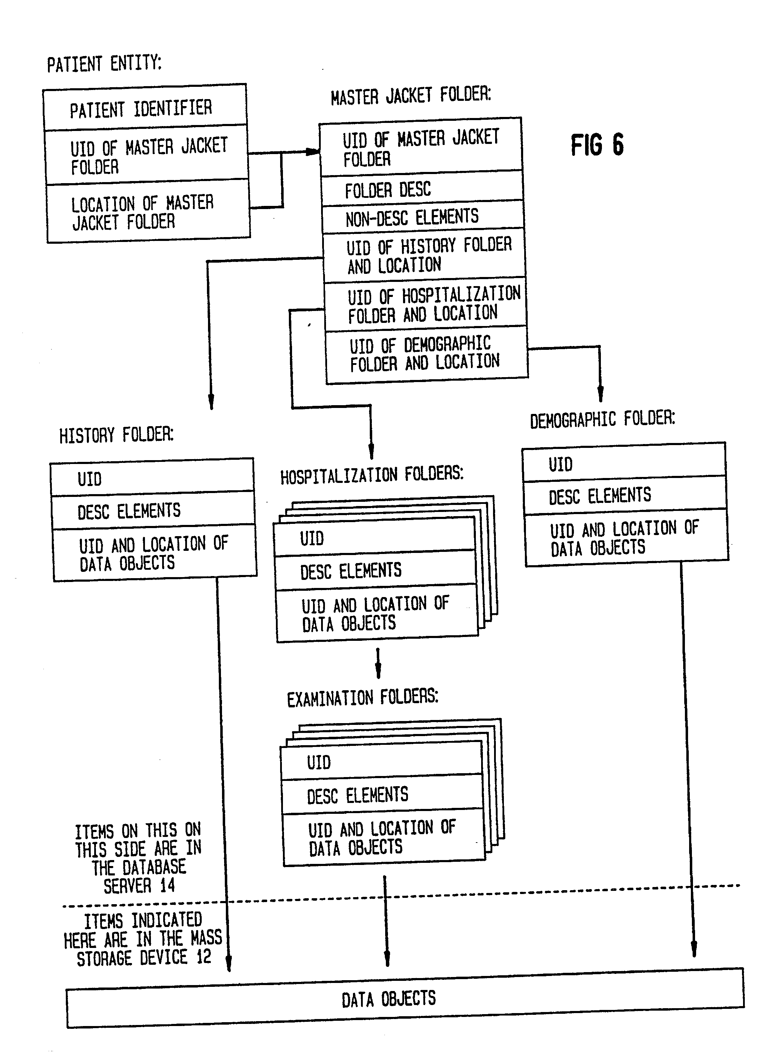

Ultimately, as is shown in Fig. 6, when for example a patient has not been hospitalized within the last year, only the Patient Entity resides in the database server 14. All other information relating to the patient is stored in the mass storage device(s) 12, perhaps on optical media.

Because each Master Jacket Folder contains information representing the last discharge date of the patient and the date that the patient's records were last accessed, it is possible to automatically purge data from the database server 14 and to store it on the mass storage device(s) 12. Further, if for example a patient is readmitted to hospital after a long period of health, the relevant folders can be retrieved from the mass storage device(s) 12 and read into the database server 14, for rapid access to all patient records.

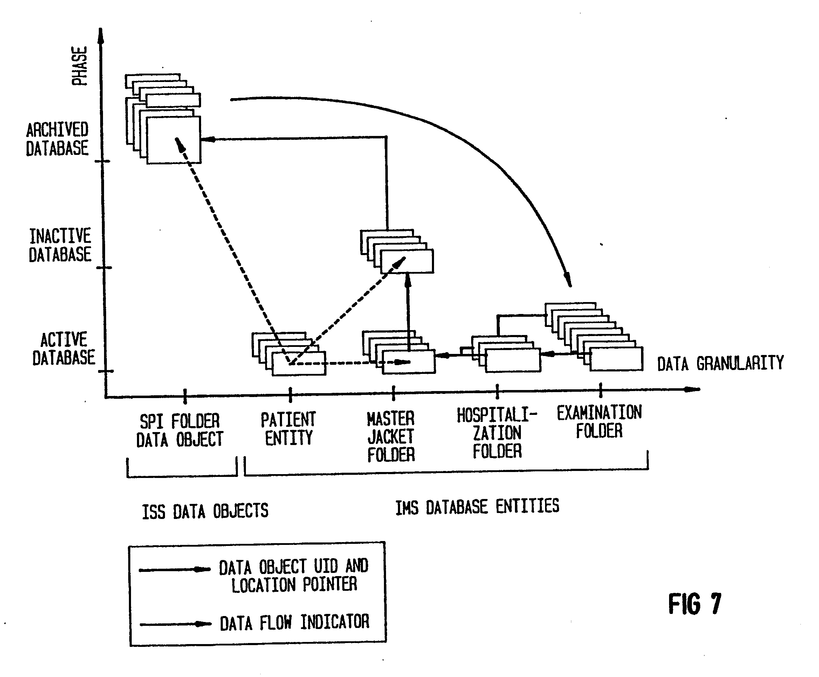

Fig. 7 illustrates how the above-described data phases relate to each other. As data ages, more and more of it is moved from the database server 14 to the mass storage device(s) 12, and less and less folders reside in the database server 14. However, when necessary, all the folders can be retrieved into the database server 14.

Advantageously, the user can define and redefine the criteria by which folders are moved from the database server 14 to the mass storage device(s) 12. For example, the above-mentioned one year period may be shortened or lengthened. Normally, folders which have been moved to the mass storage device(s) 12 will remain there until the patient has been readmitted or until they are needed for some other reason.

Those skilled in the art will understand that changes can be made in the preferred embodiments here described, and that these embodiments can be used for other purposes. Such changes and uses are within the scope of the invention, which is limited only by the claims which follow.

**Claims**

1. An information management system, comprising:
at least one mass storage means for storing data including data objects, and
one and only one database server which is operatively connected to said at least one data object storing means and which is dedicated to storage of data which is not a data object.

2. The system of claim 1, wherein
said at least one mass storage means stores data objects keyed by unique data object identifier and
said database server stores data object descriptions.

3. The system of claim 2, wherein the system manages data in a PACS.

4. A method of storing data in a PACS, comprising the following steps:
creating data using diagnostic modalities;
establishing a data object format in which a date-and-time-stamped UID is associated with DESC elements and non-DESC elements;
placing the created data into said data object format and storing said created data on at least one mass storage device;
establishing, for all created data which relates to a single patient examination, an examination folder which contains at least one UID of said created data;
establishing, for all data which relates to a single hospitalization, a hospitalization folder which contains at least one UID of an examination folder;
establishing, for data which relates to a patient's hospitalizations, a master jacket folder which contains at least one UID of a hospitalization folder;
establishing, for each master jacket folder, a patient entity which contains the UID of the master jacket folder; and
storing all patient entities on a database server which is operatively connected to said at least one mass storage device.

5. The method of claim 4, further comprising the steps of storing the examination, hospitalization and master jacket folders on said database server, evaluating said folders in accordance with predetermined criteria and moving these folders which meet said criteria to said at least one mass storage device.

6. The method of claim 4, further comprising the steps of establishing, for each patient, a history and a demographics folder, each of said master jacket folders containing a UID of a history folder and a UID of a demographics folder.

7. The method of claim 5, further comprising the steps of locating folders stored on said at least one mass storage device and moving them to the database server.

FIG 1

```
┌─────────────────────────┐
│ UID                     │
├─────────────────────────┤
│ DESC ELEMENTS           │
│                         │
│ -DATA OBJECT TYPE       │
│ -CREATION DATE          │
│            •            │
│            •            │
│            •            │
│ -OTHER DESC ELEMENTS    │
├─────────────────────────┤
│ NON-DESC                │
│ ELEMENTS                │
└─────────────────────────┘
```

**FIG 2**

PATIENT ENTITY:

```
┌─────────────────────────┐
│ PATIENT IDENTIFIER      │
├─────────────────────────┤
│ UID OF MASTER JACKET    │
│ FOLDER                  │
├─────────────────────────┤
│ LOCATION OF MASTER      │
│ JACKET FOLDER           │
└─────────────────────────┘
```

POINTS TO LOCATION
IN MASS STORAGE
DEVICE 12 OR
DATABASE SERVER

**FIG 4**

FIG 3

EP 0 380 966 A1

PATIENT ENTITY:

| PATIENT IDENTIFIER |
| UID OF MASTER JACKET FOLDER |
| LOCATION OF MASTER JACKET FOLDER |

POINTS TO LOCATION IN DATABASE SERVER

MASTER JACKET FOLDER:

| UID OF MASTER JACKET FOLDER |
| FOLDER DESC |
| NON-DESC ELEMENTS |
| UID OF HISTORY FOLDER AND LOCATION |
| UID OF HOSPITALIZATION FOLDER AND LOCATION |
| UID OF DEMOGRAPHIC FOLDER AND LOCATION |

POINTS TO LOCATION IN MASS STORAGE DEVICE 12

FIG 5

**FIG 6**

PATIENT ENTITY:

| PATIENT IDENTIFIER |
| UID OF MASTER JACKET FOLDER |
| LOCATION OF MASTER JACKET FOLDER |

MASTER JACKET FOLDER:

| UID OF MASTER JACKET FOLDER |
| FOLDER DESC |
| NON-DESC ELEMENTS |
| UID OF HISTORY FOLDER AND LOCATION |
| UID OF HOSPITALIZATION FOLDER AND LOCATION |
| UID OF DEMOGRAPHIC FOLDER AND LOCATION |

HISTORY FOLDER:

| UID |
| DESC ELEMENTS |
| UID AND LOCATION OF DATA OBJECTS |

HOSPITALIZATION FOLDERS:

| UID |
| DESC ELEMENTS |
| UID AND LOCATION OF DATA OBJECTS |

DEMOGRAPHIC FOLDER:

| UID |
| DESC ELEMENTS |
| UID AND LOCATION OF DATA OBJECTS |

EXAMINATION FOLDERS:

| UID |
| DESC ELEMENTS |
| UID AND LOCATION OF DATA OBJECTS |

ITEMS ON THIS ON THIS SIDE ARE IN THE DATABASE SERVER 14

ITEMS INDICATED HERE ARE IN THE MASS STORAGE DEVICE 12

| DATA OBJECTS |

FIG 7

PHASE

ARCHIVED
DATABASE

INACTIVE
DATABASE

ACTIVE
DATABASE

DATA GRANULARITY

SPI FOLDER
DATA OBJECT

PATIENT
ENTITY

MASTER
JACKET
FOLDER

HOSPITALI-
ZATION
FOLDER

EXAMINATION
FOLDER

ISS DATA OBJECTS

IMS DATABASE ENTITIES

DATA OBJECT UID AND
LOCATION POINTER

DATA FLOW INDICATOR

EP 0 380 966 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 206 565 (COATS VIYELLA PLC) <br> * Page 2, line 22 - page 3 * | 1,2 | G 06 F 15/42 |
| Y | | 3 | |
| Y | BIOMEDIZINISCHE TECHNIK, vol. 31, no. 6, June 1986, pages 143-149; H.U. LEMKE et al.: "Work stations for computer-graphic display in medical imaging" <br> * Pages 143-145 * | 3 | |
| Y | COMPUTERS IN CARDIOLOGY, Linkoping, 8th - 11th September 1985, pages 519-522, IEEE, New York, US; R.W. BROWER et al.: "Database management for cardiac catheterisation laboratories in the Netherlands" <br> * Page 250; figure 1 * | 4,5 | |
| A | IDEM | 6 | |
| Y | US-A-3 872 448 (B.A. MITCHELL, Jr.) <br> * Whole document * | 4,5 | TECHNICAL FIELDS SEARCHED (Int. Cl.5) <br><br> G 06 F |
| A | GB-A-2 157 036 (OLYMPUS OPTICAL CO.) <br> * Page 2, lines 55-106; page 3, line 98 - page 4, line 16 * | 1-7 | |
| Y | PROCEEDINGS PRIP 82, PATTERN RECOGNITION AND IMAGE PROCESSING, Las Vegas, 14th - 17th June 1982, pages 294-299, IEEE, New York, US; S.J. DWYER III et al.: "A diagnostic digital imaging management system" <br> * Page 294; page 295, column 2, part 2; page 296, column 2, part 2 * | 4,5 | |

The present search report has been drawn up for all claims

| Place of search | Date of completion of the search | Examiner |
|---|---|---|
| THE HAGUE | 29-03-1990 | PFITZINGER E.E. |